# EUROPEAN PATENT APPLICATION

(11) **EP 4 170 037 A1**
(43) Date of publication of application: **26.04.2023**
(21) Application number: 20941504.1
(22) Date of filing: 08.07.2020
(51) Int. Cl.: C12N 15/74, C07K 14/255, G01N 33/50, G01N 33/574, A61K 39/112, A61P 35/00

(54) **SALMONELLA STRAIN FOR TREATING CANCER AND USE THEREOF**

(30) Priority: 18.06.2020 KR 20200074197
(71) Applicant: Industry Foundation of Chonnam National University, Gwangju 61186 (KR)
(72) Inventor: MIN, Jung, Joon, Gwangju 61461 (KR); SONG, Mi, Ryung, Yongin-si Gyeonggi-do 17035 (KR); YOU, Sung, Hwan, Gwangju 61419 (KR); HONG, Yeong, Jin, Gwangju 61682 (KR); HUY, Nguyen, Dinh, Jeollanam-do, 58116 (VN)
(74) Representative: Schnappauf, Georg
(86) International application number: PCT/KR2020/008920
(87) International publication number: WO 2021/256599

(57) **Abstract**

The present invention relates to a *Salmonella* strain that selectively acts on cancer for the treatment of cancer, and a composition for preventing or treating cancer containing the same. In particular, the *Salmonella* strain according to the present invention has a tumor-suppressing effect, but has a significantly low viability in normal organs, and thus may have a significant anticancer effect compared to conventional inventions.

## Description

### Technical Field

The present invention relates to a *Salmonella* strain acting selectively on cancer and a composition for preventing or treating cancer containing the same.

### Background Art

Cancer is currently one of the diseases that cause the most deaths worldwide, and the incidence of cancer is continuously increasing due to an increase in life expectancy and a decrease in the age at which cancer occurs. According to the 2013 statistical data provided by the National Cancer Center of Korea, the total number of cancer patients in Korea registered in the Korea Central Cancer Registry in 2010 is 202,053, and tends to increase continuously.

Therefore, with respect to cancer, omnidirectional research on cancer treatment starting from basic research at the cellular level has been conducted worldwide. However, the mechanism of cancer development remains unclear, it is difficult to prevent cancer recurrence and cure cancer, and thus the demand for anticancer drugs has increased explosively, and huge research funds have been invested in research institutes and corporations. However, not only expensive cancer treatment costs that are direct medical costs, but also indirect costs due to withdrawal from social and economic activity after cancer onset, rehabilitation, and patient care, become a huge economic burden for the cancer patient's family and all members of society. Thus, there is a need to introduce a new low-cost technology.

In line with this situation, strains having an anticancer effect have been developed by many researchers since the suppression of cancer by bacteria was reported about 100 years ago, and these strains are already in clinical trials by some research groups.

The cellular environment of cancer tissue, which is represented by low partial pressure of oxygen, abundant nutrients, and lack of immune response, may be a characteristic of the habitat favored by pathogenic strains that multiply in the body. In particular, the necrotic region located in the center of cancer tissue is known to be a region to which drugs are not efficiently delivered and which cause cancer metastasis, etc. *Clostridium, Bifidobacterium, Salmonella,* etc. accumulate and multiply in abundant nutrients in the necrotic region located in the center of cancer tissue. In this process, cancer tissue shrinkage and growth inhibition are observed, and thus these strains have been used as probiotic strains for cancer treatment.

Chinese Dr. Xu's research group developed a system to deliver endostatin, an angiogenesis inhibitor, to solid cancer tissues by oral administration of *Bifidobacterium longum,* and then reported studies on cancer treatment using a mixture containing adriamycin, an anticancer antibiotic (Xu YF, et al., Cancer Gene Ther. 2007, 14(2):1517; Hu B, et al., Cancer Gene Ther. 2009, 16(8):655-63).

In addition, various research groups, including the National Cancer Institute (NCI) of the United States, reported the inhibition of cancer growth by *Salmonella,* and reported the anticancer effects of attenuated recombinant *Salmonella* strains loaded with anticancer therapeutic substances, including a mutant strain obtained by attenuating the virulence of *Salmonella typhimurium* (Nishikawa H, et al., J Clin Invest. 2006, 116(7):1946-54, Clairmont, C. et al. J.Infect. Dis. 2000, 181:1996-2002. Nguyen VH, et al., Cancer Res. 2010, 1;70(1):18-23, Kim K, et al., PLoS One. 2013, 8(3):e60511).

However, anticancer therapy using probiotic strains has been concerned about the occurrence of side effects due to the strains that also remain in normal tissues other than tumor tissues for a long time. In particular, the production of strong anticancer substances by recombinant *Salmonella* for the treatment of tumors may cause tissue damage by *Salmonella* remaining in normal tissues. In addition, when chronic infection by *Salmonella* that remains without being removed after tumor cell death occurs, the carrier may cause infection in other people. In addition, *Salmonella* with attenuated pathogenicity may cause fatal sepsis patients or old and weak people with weakened immunity, and the antibody generated during chronic infection may cause autoimmune diseases, for example, arthritis, ophthalmitis, urethritis, etc. Therefore, in the development of cancer treatment technology using *Salmonella,* there is a need to develop a *Salmonella* strain which may be rapidly cleared from normal organs upon *Salmonella* infection, by increasing the specificity of *Salmonella* to tumor tissues and the multiplication of *Salmonella* in tumor tissues and efficiently controlling the viability of *Salmonella* in normal tissues other than tumor tissues in order to minimize excessive immune responses in normal organs and side effects caused thereby. It has been reported that mutant strains with deletion of *Salmonella* pathogenicity genes modulate the virulence and viability of *Salmonella.* Inactivation of *Salmonella* pathogenicity island-1 (SPI-1) or *Salmonella* pathogenicity island-2 (SPI-2), which is a *Salmonella* pathogenicity gene cluster, showed the effect of attenuating the pathogenicity of *Salmonella gallinarum* (Korean Patent No. 1015008050000), and additional deletion of a single gene belonging to the pathogenicity gene cluster in attenuated *Salmonella* rapidly reduced the viability of the mutant strain in the spleen (Korean Patent No. 1017735800000).

However, deletion of the SPI-1 or SPI-2 gene cluster in *Salmonella galinarum* results in non-pathogenicity due to reduced penetration efficiency in avian epithelial cells, and is not considered to rapidly modulates the viability of *Salmonella* in all normal tissues *in vivo,* not immune organs, because it is impossible to confirm the viability of residual *Salmonella* in normal tissues, and the decrease in *Salmonella* viability in the spleen due to deletion of a single gene belonging to the pathogenicity gene cluster in attenuated *Salmonella* can be considered because of an effect of increasing the immune response sensitivity to *Salmonella* by the spleen, an immune organ composed of lymphocytes and various immune cells.

Accordingly, the present inventors have made efforts to develop a *Salmonella* strain whose viability in in normal tissues other than tumor tissues is efficiently controlled and which has excellent anticancer effects, and as a result, have found that, when a *Salmonella* pathogenicity gene cluster in a previously known attenuated *Salmonella* strain is additionally deleted, the resulting strain may significantly inhibit tumor growth while having reduced viability in normal organs, which demonstrates that the strain of the present invention may be effectively used as an active ingredient in an anticancer composition, thereby completing the present invention.

### DISCLOSURE

### Technical Problem

An object of the present invention is providing a *Salmonella* sp. mutant strain in which a gene is deleted, and a method for producing the same.

Another object of the present invention is to provide a composition for preventing, improving, or treating cancer containing the *Salmonella* sp. mutant strain provided by the present invention.

However, objects to be achieved by the present invention are not limited to the above-mentioned objects, and other objects not mentioned herein will be clearly understood by those of ordinary skill in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described detail in order to not unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the features, configurations, compositions, or characteristics may be combined in any suitable manner in one or more embodiments.

Unless otherwise stated in the specification, all the scientific and technical terms used in the specification have the same meanings as commonly understood by those skilled in the technical field to which the present invention pertains.

According to an embodiment of the present invention, the present invention is directed to a *Salmonella* sp. mutant strain wherein *Salmonella* pathogenicity island-1 (SPI-1) and *Salmonella* pathogenicity island-1 (SPI-2) are deleted.

In the present invention, the *Salmonella* pathogenicity island-1 (SPI-1) is a specific region in the *Salmonella* genome, which contains a type III secretion system, required for invasion of intestinal epithelial cells in the early stage of *Salmonella* infection, and a cluster of genes encoding virulent proteins (Kimbrough TG et al, Microbes Infect, (2002);4(1):75-82).

In the present invention, the *Salmonella* pathogenicity island-1 (SPI-1) may consist of the nucleotide sequence set forth in SEQ ID NO: 1.

In the present invention, the *Salmonella* pathogenicity island-2 (SPI-2) is a specific genome region containing: effector proteins which are involved in the mechanism by which *Salmonella* survives without being lysed in the phagosome after phagocytosis by macrophages in intestinal immune organs or immune organs such as the spleen and the liver after translocation across epithelial cells; and a type III secretion system that secretes these proteins (Waterman SR et al, Cell Microbiol, (2003);5(8):501-511, Abrahams GL, Cell Microbiol, (2006);8(5):728-737). The Type III secretion system (TTSS) is a pathogenic substance translocation machinery developed in Gram-negative bacteria, and is a multi-protein complex which is a syringe-type device that inject pathogenic active protein through a host cell membrane and directly into the cytoplasm (Mota LJ et al, Ann Med.(2005);37(4):234-249). It is known that the process of translocating pathogenic substances through the type III secretion system is very important in the infection route of *Salmonella* (Schlumberger MC et al, Curr Opin Microbiol, (2006);9(1):46-54). Wild-type *Salmonella* adheres to and invades the intestinal epithelial cells of the host using the TTSS protein, and survives even during phagocytosis of macrophages, and in some cases, spreads through the bloodstream, causing a systemic infection. Thus, when the TTSS protein functions normally, *Salmonella* infection progresses.

In the present invention, the *Salmonella* pathogenicity island-2 (SPI-2) may consist of the nucleotide sequence set forth in SEQ ID NO: 2.

In the present invention, the *Salmonella* sp. mutant strain of the genus *Salmonella* may be one into which a gene encoding an anticancer protein has been further introduced.

The term "anticancer protein" as used in the present invention refers to a peptide having a function capable of directly or indirectly inducing the death of cancer cells. The anticancer protein may be, for example, at least one selected from the group consisting of a toxin protein, an antibody specific for a cancer antigen or a fragment of the antibody, a tumor suppressor protein, an angiogenesis inhibitor, a cancer antigen, a prodrug-converting enzyme, and a pro-apoptotic protein, without being limited thereto.

The term "toxin protein" as used in the present invention refers to a protein that may function to induce cancer cell death directly or indirectly. For example, the toxin protein may be at least one selected from the group consisting of ricin, saporin, gelonin, momordin, debouganin, diphtheria toxin, Pseudomonas toxin, hemolysin (HlyA), FAS ligand (FASL), tumor necrosis factor-α (TNF-α), TNF-related apoptosis-inducing ligand (TRAIL), and cytolysin A (ClyA). Preferably, the toxin protein may be cytolysin A, without being limited thereto.

In the present invention, introduction of the cytolysin A gene may be performed by a recombinant vector, without being limited thereto.

In the present invention, the recombinant vector may be prepared using a forward primer represented by SEQ ID NO: 13 and a reverse primer represented by SEQ ID NO: 14.

In the present invention, the cytolysin A (ClyA) gene is a gene encoding a 34-kDa protein that lyses cell by forming a hole in the cell membrane, and it is expressed in *E. coli, Salmonella,* and the like. The sequence of the cytolysin A is described in WaiSN, Lindmark B, Soderblom T, et al. Vesicle-mediated export and assembly of pore-forming oligomers of the enterobacterial ClyA cytotoxin. Cell 2003;115:25-35, without being limited thereto, and those derived from a variety of prokaryotic cells and functional variants thereof may be used herein as the cytolysin A. In one embodiment, the protein sequence of the cytolysin A (ClyA) is available under accession number ABI83833.1, and the gene sequence thereof is available under accession number DQ910780.1. The cytolysin A gene may consist of the nucleotide sequence set forth in SEQ ID NO: 15.

The term "tumor suppressor protein" as used in the present invention refers to a gene which maintains its function in normal cells, but causes normal cells to indiscriminately divide and grow into cancer cells when the function thereof is lost. Examples of the tumor suppressor protein include, but are not limited to, retinoblastoma (RB) protein, p53 protein, adenomatous polyposis *coli* (APC) protein, phosphatase and tensin homologue (PTEN) protein, cyclin dependent kinase inhibitor 2A (CDKN2A) protein, and the like.

In the present invention, the antibody specific for cancer antigen and the fragment of the antibody is an antibody capable of specifically binding to an antigen which is a protein having a high expression level specifically on the surface or cytoplasm of a cancer cell. For example, the antibody or the fragment thereof may be an antibody specific for HER2 having a high expression level specifically in breast cancer or gastric cancer cells, without being limited thereto.

The "antibody" as used in the present invention refers to a protein molecule capable of binding specifically to an antigenic site of a protein or peptide molecule. The type of the antibody is not particularly limited, and examples thereof include polyclonal antibodies, monoclonal antibodies, or antibody fragments having an antigen-binding property, and include all types of immunoglobulin antibodies. In addition, examples of the antibody include specific antibodies such as humanized antibodies. Examples of the antibody include not only a whole antibody having two full-length light chains and two full-length heavy chains, but also a functional fragment of an antibody molecule. The "functional fragment of an antibody molecule" refers to a fragment having at least an antigen-binding function, and examples thereof include, but are not limited, Fab, F(ab'), F(ab')2, Fv, etc.

The antibody of the present invention may be produced by a conventional method after cloning the gene encoding the cancer antigen of the present invention into an expression vector according to a conventional method to obtain a protein encoded by the gene.

The term "angiogenesis inhibitor" as used in the present invention refers to a protein or compound that may function to induce the death of cancer cells directly or indirectly by inhibiting the formation of new blood vessels around cancer cells. Preferably, examples of the angiogenesis inhibitor include, but are not limited to, angiostatin, endostatin, thrombospondin, and protease inhibitory proteins.

The term "cancer antigen" as used in the present invention refers to a protein antigen which is expressed in cancer cells but is rarely expressed in normal cells, thereby inducing an anti-tumor immune response, thereby inducing the direct or indirect death of cancer cells. Preferably, the cancer antigen of the present invention may be α-fetoprotein (AFP), vascular endothelial growth factor receptor 2 (VEGFR2), Survivin, Legumain, prostate cancer-specific antigen (PCSA), or the like, without being limited thereto.

The term "prodrug-converting enzyme" as used in the present invention refers to a protein having a function of converting an inactive drug into an active drug through a metabolism caused by an enzymatic reaction. When this prodrug-converting enzyme is used, the inactive drug may be metabolized and converted into an active drug capable of directly or indirectly inducing the death of cancer cells. Thus, the prodrug-converting enzyme may be very useful for the prevention or treatment of cancer. Preferred examples of the prodrug-converting enzyme of the present invention include, but are not limited to, thymidine kinase, cytosine deaminase, nitroreductase, purine nucleoside phosphorylase, carboxypeptidase G2, chromate reductase YieF, herpes simplex virus type I thymidine kinase/ganciclovir (HSV1-TK/GCV), β-glucuronidase, and the like.

The term "pro-apoptotic protein" as used in the present invention refers to a protein that induces the direct or indirect death of cancer cells by making these cells deficient in factors (proteins, nutrients, oligonucleotides, etc.) essential for growth or maintenance of the cancer cells. Preferred examples of the pro-apoptotic protein of the present invention include, but are not limited to, L-ASNase, RNA-binding motif protein 5 (RBM5), and the like.

In the present invention, the *Salmonella* sp. mutant strain may further contain a reporter gene, and the reporter gene enables imaging of the *Salmonella* sp. mutant strain.

The reporter gene encodes a protein capable of imaging *in vivo* or *ex vivo (in vitro),* and examples thereof include bioluminescent genes, chemiluminescent genes and fluorescent genes of various origins.

In the present invention, the luminescent gene may be a luciferase gene derived from prokaryotic or eukaryotic cells. The luciferase gene derived from prokaryotic cells may be a bacterial luciferase gene (lux). The luciferase gene derived from eukaryotic cells may be a firefly-derived luminescent gene, without being limited thereto. The gene may be present in the form of a plasmid or inserted into the genome of *Salmonella.*

In the present invention, the luminescent gene may be present in the form of a plasmid or inserted into the genome of *Salmonella.*

In the present invention, the *Salmonella* sp. mutant strain may be derived from at least one selected from the group consisting of *Salmonella typhimurium, Salmonella choleraesuis, Salmonella enteritidis, Salmonella infantis, Salmonella paratyphi,* and *Salmonella typhi.* The listed *Salmonella* sp. strains are Gram-negative facultative anaerobes. They are distinguished from *E. coli* in that they have no lactose degradation ability, do not form indole, and do not produce hydrogen sulfide. The *Salmonella* sp. strains may be motile.

*Salmonella typhimurium* is a *Salmonella* sp. strain causing typhoid. *Salmonella typhimurium* is a rod-shaped bacillus with flagella and is Gram-negative. *Salmonella typhimurium* is weak to heat and dies at 60°C within 20 minutes. Livestock, wild animals, people, milk, or eggs may be primarily contaminated with *Salmonella typhimurium,* and salads, etc., which are susceptible to secondary infection from contaminated meat, may cause salmonellosis which is a kind of food poisoning.

*Salmonella choleraesuis* is a *Salmonella* sp. bacterium well known to cause swine fever pathogen and infects both humans and animals. *Salmonella choleraesuis* is a main cause of acute sepsis caused by *Salmonella.* It is a Gram-negative facultative anaerobic bacillus with peritrichous flagella, which is motile. It is distinguished from E. *coli* in that it has no lactose degradation ability, does not form indole, and does not produce hydrogen sulfide. *Salmonella choleraesuis* grows optimally at a temperature of 35 to 37°C, is capable of multiplying at a temperature of 10 to 43°C, and is killed by heating at 60°C for 20 minutes. The optimum pH for *Salmonella choleraesuis* is 7.2 to 7.4, and *Salmonella choleraesuis* has a size of 0.5 to 0.8 x 3 to 4 µm.

*Salmonella enteritidis* is a *Salmonella* sp. bacterium causing bacterial infection-type food poisoning, and is also called *Bacillus enteritidis. Salmonella enteritidis* is a representative bacterium of *Salmonella,* may cause infection in all kinds of animals, and has a very high adaptability to the host. It is a Gram-negative facultative anaerobic bacillus with peritrichous flagella, which is motile. It is distinguished from *E. coli* in that it has no lactose degradation ability, does not form indole, and does not produce hydrogen sulfide. *Salmonella enteritidis* grows optimally at a temperature of 35 to 37°C, is capable of multiplying at a temperature of 10 to 43°C, and is killed by heating at 60°C for 20 minutes. The optimum pH for *Salmonella enteritidis* is 7.2 to 7.4, and *Salmonella enteritidis* has a size of 0.5 to 0.8 x 3 to 4 µm.

*Salmonella infantis* is a bacterium that infects eggs or poultry, and *Salmonella paratyphi* and *Salmonella typhi* are bacteria causing typhoid.

In the present invention, the *Salmonella* sp. mutant strain may be a mutant strain lacking the ability to synthesize guanosine polyphosphate. The guanosine polyphosphate may be guanosine-5-diphosphate-3-diphosphate (ppGpp).

In the present invention, the *Salmonella* sp. mutant strain may be one in which at least one of ppGpp synthase-encoding *Salmonella*-relA gene and *Salmonella-spoT* gene is inactivated.

In the present invention, a *Salmonella* sp. strain in which the ppGpp synthesis gene is deleted exhibits a tumor suppressive effect, but also may exhibit significant viability even in normal organs and tissues, causing *Salmonella* infection. In addition, a strain in which only the *Salmonella* pathogenicity gene is deleted also maintains its pathogenicity in normal organs, and thus the possibility of its use as a therapeutic strain is insignificant. However, the *Salmonella* sp. gene according to the present invention, obtained by additionally deleting both the SPI-1 and SPI-2 genes from the *Salmonella* sp. gene in which the ppGpp synthesis gene is inactivated, has a significantly low viability in normal organs, and thus causes no *Salmonella* infection, and has the advantage of exhibiting a significantly better anticancer effect than the *Salmonella* sp. gene in which the ppGpp synthesis gene is deleted.

In the present invention, the *Salmonella* sp. mutant strain may be derived from SHJ2037 (accession number KCTC 10787BP).

In the present invention, the term "derived" refers to an existing plasmid or strain that has not undergone a genetic recombination process for producing a recombinant plasmid or recombinant strain.

For gene deletion and inactivation for producing the *Salmonella* sp. mutant strain of the present invention, a recombinant vector may be used in a *Salmonella* sp. host cell. The recombinant vector may be a plasmid, without being limited thereto. The plasmid may be introduced into the *Salmonella* sp. strain by an electroporation method, without being limited thereto. The electroporation method is a method in which an electric shock is applied to a protoplast so that DNA is introduced into the cell membrane. When high-voltage electrical stimulation is applied to the cell at regular intervals, a small hole in the cell membrane is opened, and at this time, DNA is inserted into the cell through this hole by the action of electrophoresis.

The *Salmonella* sp. mutant strain of the present invention may be used for the prevention or treatment of cancer.

In the present invention, the "cancer" is a disease characterized by rapid and uncontrolled growth of mutant cells, and may be at least one selected from the group consisting of melanoma, fallopian tube cancer, brain cancer, small intestine cancer, esophageal cancer, lymph adenocarcinoma, gallbladder cancer, blood cancer, thyroid cancer, endocrine adenocarcinoma, oral cancer, liver cancer, biliary tract cancer, colorectal cancer, rectal cancer, cervical cancer, ovarian cancer, kidney cancer, stomach cancer, duodenal cancer, prostate cancer, breast cancer, brain tumor, lung cancer, undifferentiated thyroid cancer, uterine cancer, colon cancer, bladder cancer, ureter cancer, pancreatic cancer, bone/soft tissue sarcoma, skin cancer, non-Hodgkin's lymphoma, Hodgkin's lymphoma, multiple myeloma, leukemia, myelodysplastic syndrome, acute lymphoblastic leukemia, acute myeloid leukemia, chronic lymphocytic leukemia, chronic myelogenous leukemia, and solitary myeloma. Preferably, the cancer may be at least one selected from the group consisting of liver cancer, biliary tract cancer, colorectal cancer, rectal cancer, cervical cancer, ovarian cancer, kidney cancer, stomach cancer, duodenal cancer, prostate cancer, breast cancer, brain tumor, lung cancer, undifferentiated thyroid cancer, uterine cancer, colon cancer, bladder cancer, ureter cancer, pancreatic cancer, bone/soft tissue sarcoma, and skin cancer. More preferably, the cancer may be colon cancer, without being limited thereto.

The *Salmonella* sp. mutant strain of the present invention may have lower viability in normal tissue than in tumor tissue. Here, the normal tissue may be a tissue of an organ selected from the group consisting of lung, liver, and spleen, without being limited thereto.

Since the *Salmonella* sp. mutant strain of the present invention has significantly lower viability in normal tissues, it causes no *Salmonella* infection and has the advantage of exhibiting a significantly better anticancer effect than conventional inventions.

In addition, it is understood that, if the *Salmonella* sp. mutant strain of the present invention is attenuated by two or more different mutations in the genome, it is safe because the possibility of the *Salmonella* sp. mutant strain returning to the parent strain is very low.

According to another embodiment of the present invention, there is provided a method for producing a *Salmonella* sp. mutant strain, the method comprising a step of removing *Salmonella* pathogenicity island-1 (SPI-1) and *Salmonella* pathogenicity island-2 (SPI-2) genes from a *Salmonella* sp. strain to obtain a transformed strain.

The *Salmonella* pathogenicity island-1 (SPI-1) in the present invention may consist of the nucleotide sequence set forth in SEQ ID NO: 1.

The *Salmonella* pathogenicity island-2 (SPI-2) in the present invention may consist of the nucleotide sequence set forth in SEQ ID NO: 2.

In the present invention, removal of the *Salmonella* pathogenic island-1 (SPI-1) and *Salmonella* pathogenic island-2 (SPI-2) genes may be performed by a recombinant vector, without being limited thereto.

In the present invention, the recombinant vector may be prepared using a forward primer represented by SEQ ID NO: 7 and a reverse primer represented by SEQ ID NO: 8.

The recombinant vector may be derived from a pKD13, pCP20 or pJL39 plasmid, without being limited thereto.

The pKD13 plasmid is 3,434 bp in length and contains beta-lactamase, rrnB transcription terminator, priming site 1, natural FRT site, common priming site kt, Tn5 neomycin phosphotransferase, common priming site k2, common priming site k1, distal 35-nt of natural FRT site, priming site 4, lambda terminator and R6K gamma replication origin gene.

The pCP20 plasmid is 9497 bp in length and contains EcoRI, cat, Pstl, HindIII Ci857, flp, bamHi, beta-lactamase, mobA, mob2 and repA101ts genes.

The production method according to the present invention may further comprise a step of introducing a gene encoding an anticancer protein.

In the present invention, the anticancer protein may be at least one selected from the group consisting of a toxin protein, an antibody specific to cancer antigen, a fragment of the antibody, a tumor suppressor protein, an angiogenesis inhibitor, a cancer antigen, a prodrug-converting enzyme, and a pro-apoptotic protein, without being limited thereto.

In the present invention, the toxin protein may be at least one selected from the group consisting of ricin, saporin, gelonin, momordin, debouganin, diphtheria toxin, Pseudomonas toxin, hemolysin (HlyA), FAS ligand (FASL), tumor necrosis factor-α (TNF-α), TNF-related apoptosis-inducing ligand (TRAIL), and cytolysin A (ClyA). Preferably, the toxin protein may be cytolysin A, without being limited thereto.

In the present invention, introduction of the cytolysin A gene may be performed by a recombinant vector, without being limited thereto. The vector used for introducing the cytolysin A gene may be the same as or different from the vector used for removing the SP-1 and SP-2 genes.

In the present invention, the recombinant vector may be prepared using a forward primer represented by SEQ ID NO: 13 and a reverse primer represented by SEQ ID NO: 14.

In the present invention, introduction of the plasmid of the recombinant vector may be performed by an electroporation method, without being limited thereto.

In the present invention, the step of introducing the cytolysin A (ClyA) gene may be performed before or after removal of the *Salmonella* pathogenicity island-1 (SPI-1) and Salmonella pathogenicity island-2 (SPI-2) genes, and the order of performing is not particularly limited.

In the present invention, the *Salmonella* sp. mutant strain may be derived from at least one selected from the group consisting of *Salmonella typhimurium, Salmonella choleraesuis, Salmonella enteritidis, Salmonella infantis, Salmonella paratyphi,* and *Salmonella typhi.*

In the present invention, the *Salmonella* sp. mutant strain may be a mutant strain lacking the ability to synthesize guanosine polyphosphate.

In the present invention, the *Salmonella* sp. strain may be one in which ppGpp synthase-encoding *Salmonella*-relA gene or *Salmonella-spoT* gene is inactivated.

The production method according to the present invention may further comprise a step of culturing the transformed strain described above.

In the present invention, the step of culturing may be performed using a lysogeny broth (LB) containing an antibiotic, and the lysogeny broth (LB) was developed by Giuseppe Bertani in order to optimize the growth and plaque formation of Shigella sp strains. The lysogeny broth (LB) generally contains peptides, casein peptones, vitamins (including vitamin B), trace elements (nitrogen, sulfur, magnesium), and minerals, and the osmotic pressure thereof is controlled by sodium chloride.

The production method according to the present invention may further comprise a step of culturing the transformed strain described above in an antibiotic medium and selecting the mutant strain.

In the present invention, the antibiotic may be kanamycin or tetracycline, without being limited thereto.

Details regarding the Salmonella sp. mutant strain of the present invention, the anticancer protein, the *Salmonella* sp. strains and the recombinant vector are the same as described above with respect to the composition, and thus repeated description thereof will be omitted to avoid excessive complexity of the present specification.

According to another embodiment of the present invention, the present invention is directed to a pharmaceutical composition for preventing or treating cancer containing, as an active ingredient, the *Salmonella* sp. mutant strain provided by the present invention.

In the present invention, the "cancer" is a disease characterized by rapid and uncontrolled growth of mutant cells, and may be at least one selected from the group consisting of melanoma, fallopian tube cancer, brain cancer, small intestine cancer, esophageal cancer, lymph adenocarcinoma, gallbladder cancer, blood cancer, thyroid cancer, endocrine adenocarcinoma, oral cancer, liver cancer, biliary tract cancer, colorectal cancer, rectal cancer, cervical cancer, ovarian cancer, kidney cancer, stomach cancer, duodenal cancer, prostate cancer, breast cancer, brain tumor, lung cancer, undifferentiated thyroid cancer, uterine cancer, colon cancer, bladder cancer, ureter cancer, pancreatic cancer, bone/soft tissue sarcoma, skin cancer, non-Hodgkin's lymphoma, Hodgkin's lymphoma, multiple myeloma, leukemia, myelodysplastic syndrome, acute lymphoblastic leukemia, acute myeloid leukemia, chronic lymphocytic leukemia, chronic myelogenous leukemia, and solitary myeloma. Preferably, the cancer may be at least one selected from the group consisting of liver cancer, biliary tract cancer, colorectal cancer, rectal cancer, cervical cancer, ovarian cancer, kidney cancer, stomach cancer, duodenal cancer, prostate cancer, breast cancer, brain tumor, lung cancer, undifferentiated thyroid cancer, uterine cancer, colon cancer, bladder cancer, ureter cancer, pancreatic cancer, bone/soft tissue sarcoma, and skin cancer. More preferably, the cancer may be colon cancer, without being limited thereto.

The term "preventing" as used in the present invention may include, without limitation, any action that blocks symptoms caused by cancer or suppresses or delays the symptoms, by using the composition of the present invention.

The term "ameliorating" or "treating" as used in the present invention may include, without limitation, any action that beneficially changes symptoms caused by cancer or benefits an individual having the symptoms, by using the active ingredient of the present invention.

The pharmaceutical composition of the present invention may be in the form of capsules, tablets, granules, injections, ointments, powders or beverages, and the pharmaceutical composition may be for administration to humans.

For use, the pharmaceutical composition of the present invention may be formulated in the form of, but not limited to, oral dosage forms such as powders, granules, capsules, tablets, aqueous suspensions, etc., external preparations, suppositories, and sterile injection solutions, according to the respective conventional methods. The pharmaceutical composition of the present invention may contain pharmaceutically acceptable carriers. Pharmaceutically acceptable carriers that may be used for oral administration include binders, lubricants, disintegrants, excipients, solubilizers, dispersants, stabilizers, suspending agents, pigments, flavorings, and the like, and pharmaceutically acceptable carriers that may be used for injection include buffers, preservatives, analgesics, solubilizers, isotonic agents, stabilizers, and the like. Pharmaceutically acceptable carriers that may be used for topical administration include bases, excipients, lubricants, preservatives, and the like. The dosage forms of the pharmaceutical composition of the present invention may be prepared in various ways by mixing with pharmaceutically acceptable carriers as described above. For example, for oral administration, the pharmaceutical composition may be prepared in the form of tablets, troches, capsules, elixir, suspensions, syrups, wafers, and for injection, the pharmaceutical composition may be presented in unit dose ampoules or multi-dose containers. In addition, the pharmaceutical composition may be formulated as solutions, suspensions, tablets, capsules, sustained-release preparations, or the like.

Examples of carriers, excipients, and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyl-hydroxy benzoate, propyl-hydroxy benzoate, talc, magnesium stearate, and mineral oil. In addition, the pharmaceutical composition may further contain a filler, an anticoagulant, a lubricant, a wetting agent, a fragrance, an emulsifier, a preservative, or the like.

The routes of administration of the pharmaceutical composition according to the present invention include, but are not limited to, oral, intravenous, intramuscular, intraarterial, intramedullary, intradural, intracardiac, transdermal, subcutaneous, intraperitoneal, intranasal, gastrointestinal, topical, sublingual and intrarectal routes. Oral or parenteral administration is preferred.

In the present invention, "parenteral" includes subcutaneous, transdermal, intravenous, intramuscular, intra-articular, intra-synovial, intrasternal, intradural, intralesional and intra-cranial injection or infusion techniques. The pharmaceutical composition of the present invention may also be formulated as suppositories for intrarectal administration.

The pharmaceutical composition of the present invention may vary depending on various factors, including the activity of a specific compound used, the patient's age, body weight, general health, sex, diet, the time of administration, the route of administration, excretion rate, the drug content, and the severity of a specific disease to be prevented or treated. The dose of the pharmaceutical composition may be suitably selected by a person skilled in the art depending on the patient's condition, body weight, the severity of the disease, the form of drug, and the route and period of administration, and may be 0.0001 to 50 mg/kg/day or 0.001 to 50 mg/kg/day. The pharmaceutical composition may be administered once or several times a day. The dose is not intended to limit the scope of the present invention in any way. The pharmaceutical composition according to the present disclosure may be formulated as pills, sugar-coated tablets, capsules, liquids, gels, syrups, slurries, or suspensions.

In another embodiment of the present invention, there is provided a composition for diagnosing cancer containing the Salmonella sp. mutant strain as an active ingredient.

The diagnostic composition of the present invention contains the strain according to the present invention as an active ingredient.

After the mutant strain according to the present invention targets cancer cells in an individual, a reporter protein capable of imaging in real time in the strain is expressed. Thus, the strain may diagnose cancer in real time without noise signals for normal tissues.

The term "reporter protein" as used in the present invention refers to a protein that functions so that cancer can be visually diagnosed. For example, the reporter protein may be, but is not limited to, at least one selected from the group consisting of a fluorescent protein, luciferase, and a protein that is used in nuclear medicine or MRI imaging.

The term "fluorescent protein" as used in the present invention is a protein that fluoresces by itself so that cancer can be visually diagnosed. For example, the fluorescent protein may be at least one selected from the group consisting of green fluorescent protein (GFP), modified green fluorescent protein (MGFP), enhanced green fluorescent protein (EGFP), red fluorescent protein (RFP), enhanced red fluorescent protein (ERFP), blue fluorescent protein (BFP), enhanced blue fluorescent protein (EBFP), yellow fluorescent protein (YFP), and enhanced yellow fluorescent protein (EYFP), without being limited thereto.

In the present invention, the protein that is used in nuclear medicine or MRI imaging may be, for example, at least one selected from the group consisting of herpes simplex virus thymidine kinase, dopamine receptor, somatostatin receptor, sodium-iodide transporter, iron receptor, transferrin receptor, ferritin, and iron transporter (magA), without being limited thereto.

The term "diagnosing" as used in the present invention refers to any action that detects cancer tissue *in vivo,* including monitoring the presence of cancer in real time by the reporter protein expressed from the DNA construct introduced into the strain, when the strain of the present invention is located by targeting cancer.

In the diagnostic composition of the present invention, details regarding the mutant strain, vector, transformation, cancer, etc. are the same as described above, and thus repeated description thereof will be omitted to avoid excessive complexity of the present specification.

In another embodiment of the present invention, there is provided a method for providing information for diagnosing cancer comprising a step of treating a biological sample with the *Salmonella* sp. mutant strain.

The method of the present invention comprises a step of treating a biological sample, isolated from a subject of interest, with the mutant strain according to the present invention.

The method for providing information for diagnosing cancer according to the present invention may further comprises a step of diagnosing cancer when a reporter protein is expressed from the strain.

The term "biological sample" as used in the present invention refers to any material, tissue or cell obtained or derived from the subject. Examples of the biological sample include, but are not limited to, tissues, cells, or cell extracts.

In the method for providing information for diagnosis according to the present invention, details regarding the mutant strain, anticancer protein, reporter protein, vector, strain, transformation, cancer, diagnosis, etc., are the same as described above with respect to the reporter protein, the vector, the strain, and the pharmaceutical composition for preventing or treating cancer, and thus repeated description thereof will be omitted in order to avoid excessive complexity of the present specification.

Another embodiment of the present invention is directed to a method for preventing or treating cancer comprising a step of administering to a subject an effective amount of the *Salmonella* sp. mutant strain provided by the present invention.

The term "subject" as used in the present invention refers to an individual in need of prevention or treatment of cancer, and may include both mammals and non-mammals. Here, examples of the mammals include, but are not limited to, humans, non-human primates such as chimpanzees, or other ape or monkey species; farm animals such as cattle, horses, sheep, goats, swine; domestic animals such as rabbits, dogs, or cats; laboratory animals including rodents, such as rats, mice, and guinea pigs, or the like. In the present invention, examples of the non-mammals include, but are not limited to, birds or fish.

The term "administering" as used herein refers to the process of introducing the active ingredient of the present invention to the subject by any suitable method. The dosage form of the *Salmonella* sp. mutant strain that is administered as described above is not particularly limited, and the mutant strain may be administered as preparations in solid form, preparations in liquid form, or aerosol preparations for inhalation, or may be administered as preparations in solid form which are intended to be converted, immediately before use, into preparations in liquid form, for oral or parenteral administration. For example, the pharmaceutical composition of the present invention may be formulated for administration in the form of, but not limited to, oral preparations, such as powders, granules, capsules, tablets, and aqueous suspensions, as well as external preparations, suppositories, and sterile injectable solutions.

In addition, in the present invention, pharmaceutically acceptable carriers may be additionally administered together with the *Salmonella* sp. mutant strain of the present invention. Here, examples of pharmaceutically acceptable carriers that may be used for oral administration include binders, lubricants, disintegrants, excipients, solubilizers, dispersants, stabilizers, suspending agents, colorants, flavorings, and the like, and examples of pharmaceutically acceptable carriers that may be used for injection include buffers, preservatives, analgesics, solubilizers, isotonic agents, stabilizers, and the like. Examples of pharmaceutically acceptable carriers that may be used for topical administration include bases, excipients, lubricants, preservatives, and the like. The dosage form of the *Salmonella* sp. mutant strain of the present invention may be prepared in various ways by mixing with the aforementioned pharmaceutically acceptable carriers. For example, for oral administration, the pharmaceutical composition may be prepared in the form of tablets, troches, capsules, elixir, suspensions, syrups, wafers, and for injection, the pharmaceutical composition may be prepared in a unit dose form or prepared to be contained in a multi-dose container. In addition, the pharmaceutical composition may be formulated as solutions, suspensions, tablets, capsules, sustained-release preparations, or the like.

Meanwhile, examples of carriers, excipients, and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyl-hydroxy benzoate, propyl-hydroxy benzoate, talc, magnesium stearate, and mineral oil. In addition, the pharmaceutical composition may further contain a filler, an anticoagulant, a lubricant, a wetting agent, a fragrance, an emulsifier, a preservative, or the like.

The routes of administration of the *Salmonella* sp. mutant strain according to the present invention include, but are not limited to, oral, intravenous, intramuscular, intraarterial, intramedullary, intradural, intracardiac, transdermal, subcutaneous, intraperitoneal, intranasal, gastrointestinal, topical, sublingual and intrarectal routes. Oral or parenteral administration is preferred.

In the present disclosure, "parenteral" includes subcutaneous, transdermal, intravenous, intramuscular, intra-articular, intra-synovial, intrasternal, intradural, intralesional and intra-cranial injection or infusion techniques. The pharmaceutical composition of the present disclosure may also be formulated as suppositories for intrarectal administration.

In the present invention, the "effective amount" refers to a sufficient amount of an agent to provide a desired biological result. That result can be reduction and/or alleviation of a sign, symptom, or cause of a disease or disorder, or any other desired alteration of a biological system. For example, an effective amount for therapeutic uses is the amount of the *Salmonella* sp. mutant strain disclosed herein required to provide a clinically significant reduction in disease. An appropriate "effective" amount in any individual case may be determined by one of ordinary skill in the art using routine experimentation. Accordingly, the expression "effective amount" generally refers to an amount in which an active substance has a therapeutic effect. In the case of the present invention, the active substance is an agent for preventing, ameliorating, or treating cancer.

The *Salmonella* sp. mutant strain of the present invention may vary depending on various factors, including the activity of a specific compound used, the patient's age, body weight, general health, sex, diet, the time of administration, the route of administration, excretion rate, the drug content, and the severity of a specific disease to be prevented or treated. The dose of the *Salmonella* sp. mutant strain may be suitably selected by a person skilled in the art depending on the patient's condition, body weight, the severity of the disease, the form of drug, and the route and period of administration, and may be 0.0001 to 100 mg/kg/day or 0.001 to 100 mg/kg/day. The *Salmonella* sp. mutant strain may be administered once or several times a day. The dose is not intended to limit the scope of the present invention in any way. The *Salmonella* sp. mutant strain according to the present invention may be formulated as pills, sugar-coated tablets, capsules, liquids, gels, syrups, slurries, or suspensions.

In the present invention, details regarding the cancer are the same as described above, and thus detailed description thereof will be omitted to avoid excessive complexity of the present specification.

The *Salmonella* sp. mutant strain of the present invention may be used alone or in combination with methods using surgery, radiotherapy, hormone therapy, chemotherapy, and biological response modifiers.

### Advantageous Effects

The gene-deleted *Salmonella* sp. mutant strain according to the present invention has very low viability in normal tissues, but effectively induces the death of cancer cells while surviving in cancer tissues, thereby exhibiting excellent anticancer effects, and has the advantage of causing no *Salmonella* infection, unlike *Salmonella* sp. strains.

### Brief Description of Drawings

FIG. 1 is a schematic view showing a method for constructing a pJH18 plasmid.
FIG. 2 is a graph showing the growth rates of recombinant strains containing the pJH18 plasmid.
FIG. 3 is a graph showing the growth rates of strains.
FIG. 4 shows the results of analyzing the expression of the reporter protein of each *Salmonella* strain.

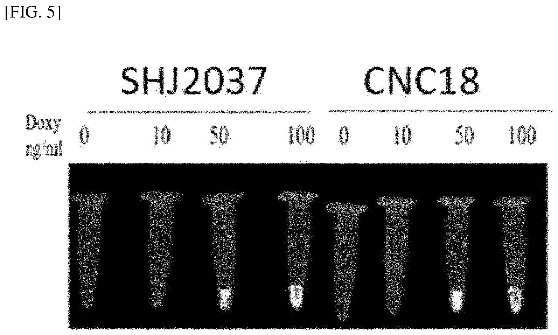

FIG. 5 shows the results of imaging performed by adding coelenterazine to measure the luciferase activity in each cultured strain.
FIG. 6 is a graph showing the luciferase activity in each cultured strain.
FIG. 7 shows the results of measuring the degree of hemolytic activity of a strain against blood agar.
FIG. 8 is a graph showing the results of measuring ATP in cultures according to an example of the present invention.
FIG. 9 shows the results of analyzing the distribution of *Salmonella* in a mouse liver.
FIG. 10 shows the results of analyzing the distribution of *Salmonella* in a mouse tumor.
FIG. 11 shows a process of extracting a spleen from each strain-treated tumor animal model.
FIG. 12 shows the results of measuring the size of the spleen of each strain-treated tumor animal model.
FIG. 13 is a graph showing the results of measuring the weight of the spleen of each strain-treated tumor animal model.
FIG. 14 is a graph showing the tumor volume measured after inoculation of a CT26-injected tumor animal model with each of CNC18 as an embodiment of the present invention and PBS and SHJ2037 as controls.
FIG. 15 is a graph showing the results of analyzing the viability after inoculation of each strain in the CT26-injected tumor animal model.
FIG. 16 is a graph showing the tumor volume measured after inoculation of an MC38-injected tumor animal model with CNC18 as an embodiment of the present invention and PBS and SHJ2037 as controls.
FIG. 17 is a graph showing the results of analyzing the viability after inoculation of each strain in the MC38-injected tumor animal model.
FIG. 18 is a graph showing the results of measuring and comparing the proportion of leukocytes in a tumor after injecting each strain into a tumor animal model.
FIG. 19 is a graph showing the results of measuring and comparing the proportion of neutrophils in a tumor after injecting each strain into a tumor animal model.
FIG. 20 is a graph showing the results of measuring and comparing the proportion of natural killer cells in a tumor after injecting each strain into a tumor animal model.
FIG. 21 is a graph showing the results of measuring and comparing the proportion of CD8+ T cells in a tumor after injecting each strain into a tumor animal model.
FIG. 22 is a graph showing the results of measuring and comparing the proportion of dendritic cells in a lymph node after injecting each strain into a tumor animal model.
FIG. 23 is a graph showing the results of measuring and comparing the proportion of M2 macrophages in a tumor after injecting each strain into a tumor animal model.
FIG. 24 is a graph showing the results of measuring and comparing the proportion of regulatory T (Treg) cells in a tumor after injecting each strain into a tumor animal model.
FIG. 25 shows images obtained using an *in vivo* imaging system (IVIS) for tumors and organs extracted on day 1 after injection of each of SHJ2037lux and CNC181ux strains into a tumor animal model.
FIG. 26 shows images obtained using an *in vivo* imaging system (IVIS) for tumors and organs extracted on day 3 after injection of each of SHJ2037lux and CNC181ux strains into a tumor animal model.
FIG. 27 shows images obtained using an *in vivo* imaging system (IVIS) for tumors and organs extracted on day 5 after injection of each of SHJ2037lux and CNC 181ux strains into a tumor animal model.
FIG. 28 shows images obtained using an *in vivo* imaging system (IVIS) after injection of each of SHJ2037lux and CNC18lux strains into a multiple myeloma animal model.
FIG. 29 shows images obtained using an *in vivo* imaging system (IVIS) after injection of each of SHJ2037lux and CNC181ux strains into a multiple myeloma animal model.

### Best Mode

According to an embodiment of the present invention, the present invention is directed to a *Salmonella* sp. mutant strain in which *Salmonella* pathogenicity island-1 (SPI-1) and *Salmonella* pathogenicity island-2 (SPI-2) are deleted.

### Mode for Invention

Hereinafter, the present invention will be described in more detail with respect to examples. These examples are only for explaining the present invention in more detail, and it will be obvious to those of ordinary skill in the art that the scope of the present invention according to the subject matter of the present invention is not limited by these examples.

### [Preparation Example 1] Construction of attenuated Salmonella

An attenuated *Salmonella* strain of the present invention was constructed by using the SHJ2037 strain as a template and deleting a pathogenicity-related gene or gene cluster by homologous recombination using lambda (λ) phage.

Specifically, a DNA fragment having a Kan cassette at the position of the open reading frame of each gene was amplified by PCR using the pKD13 plasmid. The PCR primer sequences used for removal of a total of three genes (clusters) are shown in Table 1 below. The PCR product pKD46 was introduced into a Salmonella typhimurium SMR2130 (SHJ2037, DrelA, DspoT) suspension containing cells by an electroporation method, and colonies were selected by culturing in a kanamycin medium. Then, a pCP20 plasmid (Datsenko KA, et al., Proc Natl Acad Sci US A. 2000, 6;97(12):6640-5) was introduced into the transformed strains by electroporation, and the kan cassette was removed by FLP recombinase, thereby constructing strains.

**[Table 1]**

| Mutant strain | Gene | Primer sequence (forward: 5' → 3') | Primer sequence (reverse: 5' → 3') |
|---|---|---|---|
| CNC16 | hilD | SEQ ID NO: 3 | SEQ ID NO: 4 |
| CNC17 | SPI 1 | SEQ ID NO: 5 | SEQ ID NO: 6 |
| CNC18 | SPI 1 and 2 | SEQ ID NO: 7 | SEQ ID NO: 8 |

### [Preparation Example 2] Construction of recombinant Salmonella for gene expression

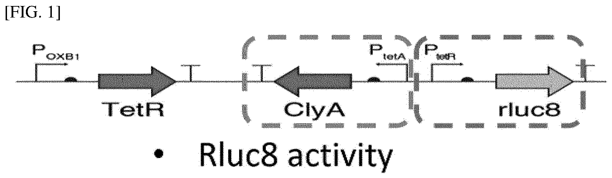

FIG. 1 is a schematic view showing a method for constructing a pJH18 plasmid. Using a pJL39 plasmid *(*Mol Ther., 21(11), p. 1985-1995, (2013)) as a template strand, a tetR gene was amplified using a forward primer (5'-CGGAATTCACCATGTCTAGATTAGATAAAAGTAAAGTGATTAACAG-3'; SEQ ID NO: 9) constructed to contain the restriction enzyme EcoRI site and a reverse primer (5'-GCTCTAGACAGCTGTTAAGACCCACTTTCACATTTAAGTTGTTTTTCT-3'; SEQ ID NO: 10) constructed to contain the restriction enzyme PvuII-Xbal site. Next, the amplification product was cleaved with the restriction enzymes EcoRI and XbaI and purified to obtain a tetR gene amplification product which was then introduced into a pBAD24 (Catalog No. ATCC^{®} 87399^{™}, ATCC, USA) plasmid, thereby constructing a pBAD-TetR plasmid.

Thereafter, through PvuII and HindIII fragments of the pJL39 plasmid, a divergent promoter region containing a multiple cloning site was introduced into the pBAD-TetR plasmid, thereby constructing a pTetR-BAD plasmid. Using NheI and Pcil restriction enzymes, the araC and araBAD promoters were removed from the pTetR-BAD plasmid, thereby constructing a pTetIIplasmid.

In addition, the constitutive promoter OXB1, obtained by amplification using pSF-OXB1 (Oxford Genetics, England) as a template and a forward primer (5'-CTACTCCGTCAAGCCGTCAAGCTGTTGTGACCGCTTGCT-3'; SEQ ID NO: 11) and a reverse primer (5'-TGAATTCCTCCTGCTAGCTAGTTGGTAACGAATCAGACGCCGGGTAATACCG GATAG-3'; SEQ ID NO: 12), was introduced into the pTetII plasmid by the Gibson assembly method, thereby constructing a pJH18 plasmid comprising the OXB1, tetA and tetR promoters.

Using the pJH18 plasmid as a template, the genes encoding Rluc8 and cytolysin A (ClyA) were introduced downstream of the promoters in the combination shown in FIG. 1, thereby constructing a pJH18-CR plasmid. Here, the cytolysin A (ClyA) gene was introduced using a forward primer (5'-AGTCCATGGTTATGACCGGAATATTTGC-3' (SEQ ID NO: 13)) and a reverse primer (5'-GATGTTTAAACTCAGACGTCAGGAACCTC-3' (SEQ ID NO: 14)).

### [Preparation Example 3] Construction of transformed Salmonella sp. mutant strain

The plasmid constructed in Preparation Example 2 was transformed by electroporation into the Salmonella strains constructed in Preparation Example 1, and then each of the transformed strains was cultured overnight using a lysogeny broth (LB) solid medium containing 100 µg/ml of ampicillin. Thereafter, the resulting colonies were cultured in LB liquid media containing ampicillin and used in the experiment. Table 2 below summarizes the transformed contents in the *Salmonella* sp. mutant strains constructed as described above.

**[Table 2]**

| | Mutant strain | Deleted genes | Inserted genes |
|---|---|---|---|
| Comparative Example 1 | SHJ2037 | relA, spoT | ClyA, Rluc8 |
| Comparative Example 2 | CNC16 | relA, spoT, hilD | ClyA, Rluc8 |
| Comparative Example 3 | CNC17 | relA, spoT, SPI 1 | ClyA, Rluc8 |
| Preparation Example | CNC18 | relA, spoT, SPI 1, 2 | ClyA, Rluc8 |

### [Experimental Example 1] Evaluation of protein expression and activity of recombinant Salmonella

### [1-1] Comparison of growth between of Salmonella mutant strains and existing strains

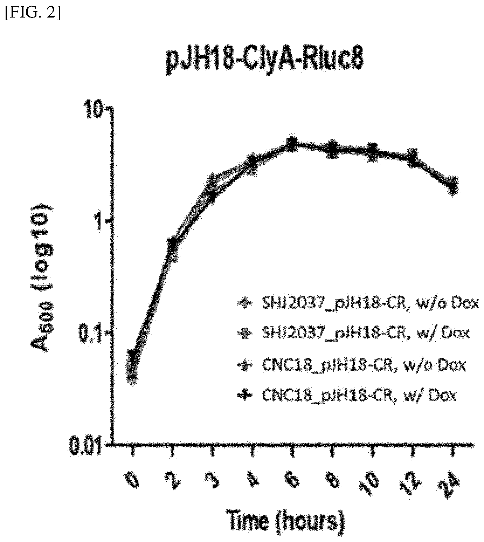

Each of the recombinant SHJ2037 and CNC18 colonies prepared in Comparative Example 1 and the Preparation Example was grown overnight in an LB liquid medium containing ampicillin, and then diluted at a ratio of 1:100 with a fresh LB medium and further cultured. When the OD₆₀₀ value reached 0.5 to 0.7, doxycycline diluted with ethanol to a final concentration of 200 ng/ml was added to the cultures which were then cultured in a shaking incubator under conditions of 200 rpm and 37°C. The growth patterns of the strains were analyzed by measuring the OD₆₀₀ value at different culture time points, and the results are shown in FIG. 2.

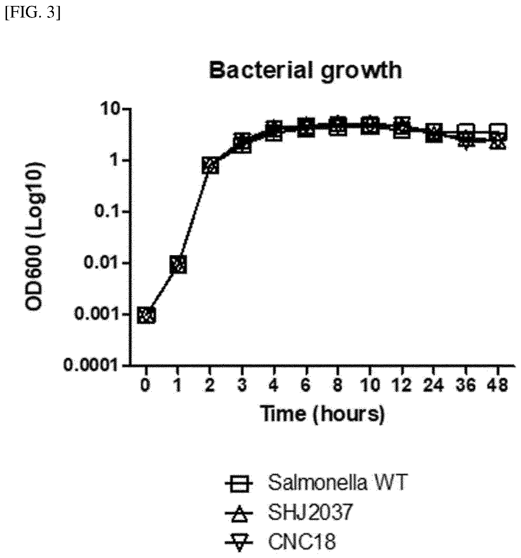

Meanwhile, the non-recombinant existing *Salmonella* colonies were treated in the same way as described above, and the results are shown in FIG. 3.

As shown in FIGS. 2 and 3, recombinant *Salmonella* CNC18 had little difference in growth rate from the existing attenuated *Salmonella* SHJ2037 and the wild-type *Salmonella* strain, and the growth thereof was not particularly inhibited even during protein expression using doxycycline. Therefore, it was confirmed that the deletion of the pathogenicity genes in the *Salmonella* strain constructed as described above did not affect the growth and gene expression of the *Salmonella* strain.

### [1-2] Comparison of protein expression levels by Western blot analysis

In order to compare the expression level of the cytolysin A (ClyA) gene between the recombinant *Salmonella* strains SHJ2037 and CNC18 constructed in Comparative Example 1 and the Preparation Example, expression of Rluc8 protein in the strains cultured as described in Experimental Example [1-1] was analyzed by Western blot analysis using an antibody specific to the protein.

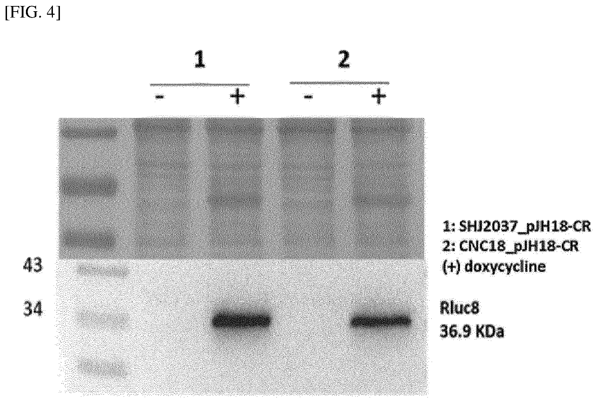

Specifically, the cultures of each strain cultured in Experimental Example [1-1] was diluted with PBS to a concentration of 4×10⁷ CFU/ml and centrifuged at 13,000 rpm for 5 minutes, and the pellet fraction was collected. The pellet fraction was washed with PBS and mixed with an SDS sample buffer containing 0.2% β-mercaptoethanol (Catalog No. EBA-1052. ELPIS BIOTECH) to obtain a strain lysate. Thereafter, the strain lysate was electrophoresed on 12% SDS-PAGE gel, and the protein on the gel was transferred to a nitrocellulose membrane and blocked with 5% skim milk at room temperature. Thereafter, the expression level of the Rluc8 protein was analyzed using Rluc8 antibody (Catalog No. AB3256, Millipore, USA), and the results are shown in FIG. 4.

As shown in FIG. 4, it was confirmed that the two recombinant *Salmonella* strains SHJ2037 and CNC18 overexpressed the Rluc8 gene, and there was no significant difference in the expression level of the Rluc8 gene between the strains.

### [1-3] Comparison of functional expression level of protein by activity assay

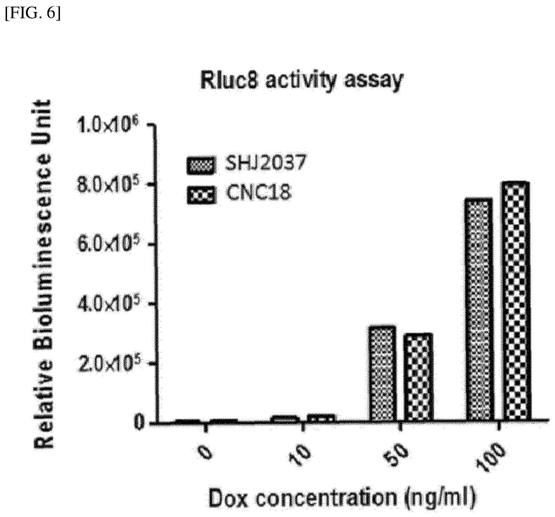

In order to measure the luciferase activity in the strains cultured in Experimental Example 1, each of the strains was resuspended in 1 ml of PBS. Next, 1 µg/ml of the substrate coelenterazine diluted in ethanol was added to the resuspended strain, and then the luciferase activity value in the strain was measured for an exposure time of 1 second using NightOWL II LB 983 In Vivo imaging system (Berthold technologies, GmbH & Co. KG, Germany) or Biorad Imager ChemoDoc^{™} XRS+ system. The measured value was normalized by the CFU of each strain, and the normalized value was calculated as relative luminescence units (RLUs), and the results are shown in FIG 6.

As shown in FIG. 6, it was confirmed that the luciferase activity value was found only in the presence of doxycycline, and sensitivity to doxycycline and the maximum activity value were similar between the strains.

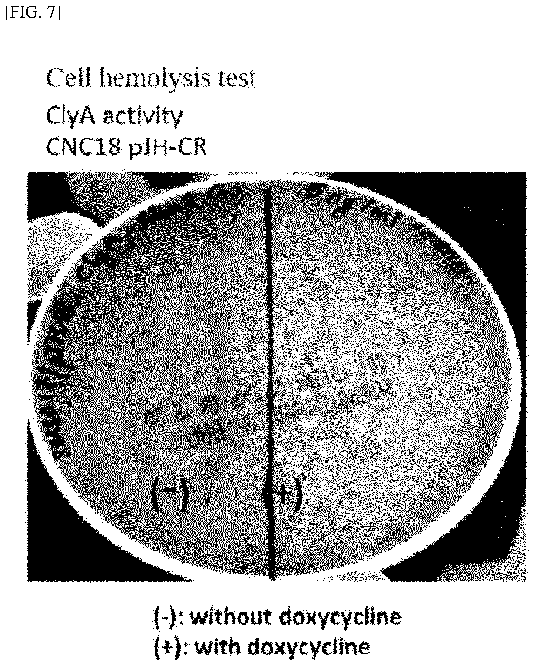

In addition, the PBS-diluted recombinant *Salmonella* strain selected in Preparation Example 3 was plated on a blood agar plate containing 0 or 20 ng/ml of doxycycline, and cultured overnight at 37°C, and the plates were imaged. The results are shown in FIG. 7.

As shown in FIG. 7, it was confirmed that the blood agar hemolytic activity of the strain appeared only in the case in which doxycycline for the gene encoding cytolysin A was included (+).

From the above results, it is confirmed that the recombinant *Salmonella* strain CNC18 according to the present invention can regulate cytolysin A (ClyA) gene expression and functions as an anticancer gene vehicle.

### [Experimental Example 2] Examination of release of DAMP signals from tumor cells by recombinant Salmonella treatment

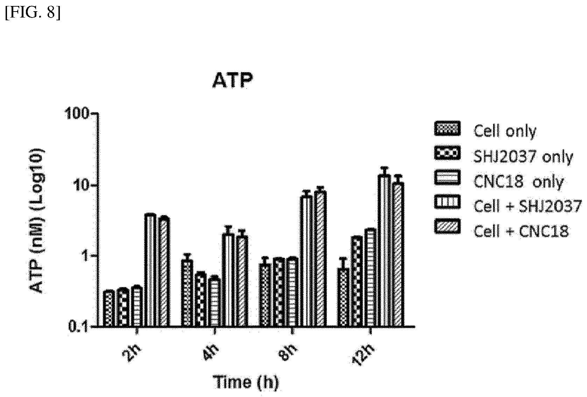

In order to evaluate the effects of the recombinant *Salmonella* strains on tumor cells, changes in damage-associated molecular patterns (DAMP) that can cause an immune response in the tumor cells treated with each of the SHJ2037 and CNC18 strains were examined. Among the DAMPs, ATP that is released extracellularly was measured. Specifically, the mouse CT26 colon cancer cell lines CRL-2638 and HB-8064 (ATCC, USA) were cultured with high-glucose DMEM (Dulbecco's Modified Eagles Medium) medium (catalog number: #LM 001-05, Welgene, Korea) containing 10% fetal bovine serum (FBS) and 1% penicillin-streptomycin in a 5% CO₂ incubator at 37°C, and then the tumor cells were treated and co-cultured with the recombinant *Salmonella.* The ATP from each of the cultures was measured using an assay kit at different time points, and the results are shown in FIG. 8.

As shown in FIG. 8, it was confirmed that the ATP released from the tumor cells increased in the case of *Salmonella* treatment, indicating that the *Salmonella* strain itself according to the present invention may be used for tumor treatment.

### [Experimental Example 3] Analysis of distribution of recombinant Salmonella in small animal tumor models

The attenuated Salmonella strain was injected into small animal mouse models, which are used in research on anticancer treatment, and the growth rate and distribution of the strain in the host were examined.

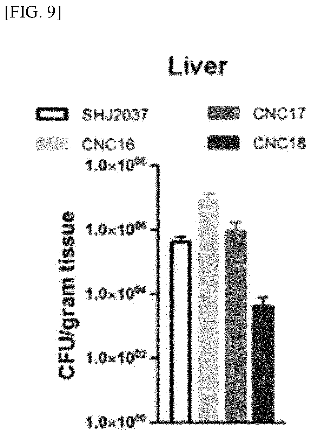

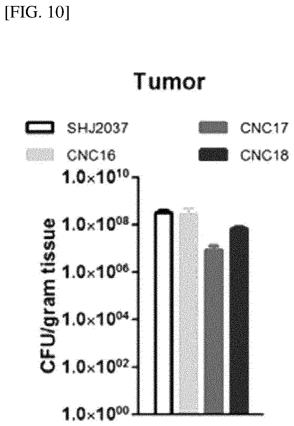

Specifically, four kinds of strains (SHJ2037, CNC16, CNC17, and CNC18) were each cultured in LB liquid medium, and then washed and diluted with PBS. Each of the strains was injected into the tail vein of each mouse to a final strain concentration is 2 x 10⁷ CFU / mouse, and 3 mice per strain were euthanized. Then, the organs of the host were extracted, minced, and diluted. Each dilution was plated and incubated on solid LB medium, and viable cells were counted. The results are shown in FIG. 9. In addition, mice bearing tumors were treated in the same way as described above, the tumors of the host were extracted, minced, and diluted, and plated and incubated on solid LB medium, viable cells were counted, and the results are shown in FIG. 10.

As a result, as shown in FIGS. 9 and 10, it was confirmed that the strain CNC18 died rapidly while showing very low viability in the liver, but remained in the tumor, indicating that it has a specific and selective targeting ability.

On the other hand, as shown in FIG. 9, it was confirmed that the strain CNC16 strain had a higher viability than the strain SHJ2037 in the liver, and had at least 100 times higher viability than the strain CNC18. That is, it was confirmed that the strain CNC16 has non-specific targeting ability in organs other than tumors.

In addition, CNC17 also showed higher viability than the strain SHJ2037 in the liver, indicating that the strain CNC17 also has non-specific targeting ability.

As shown in FIG. 10, the strain CNC17 had at least 10 times lower viability than the strain CNC18 in the tumor. That is, it was confirmed that the strain CNC17 had a lower effect than the strain CNC18 against tumors.

### [Experimental Example 4] Analysis (1) of hyperinflammatory response upon Salmonella injection

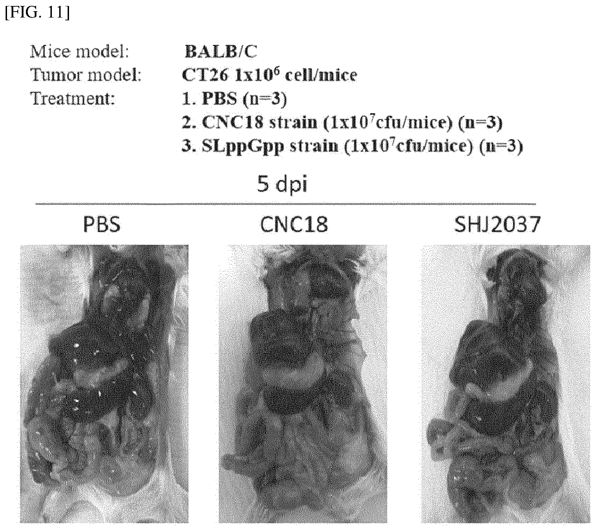

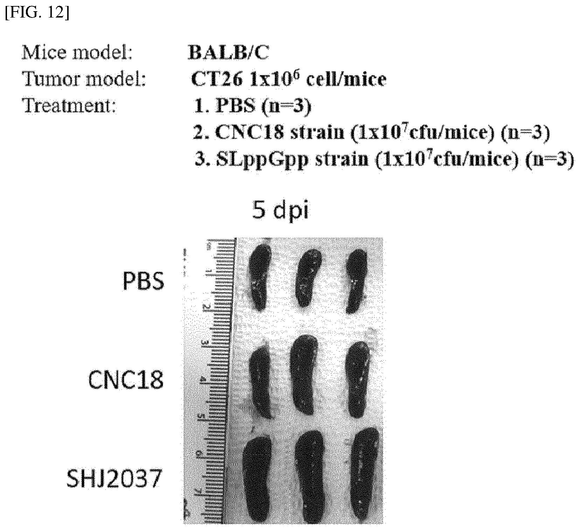

First, in order to examine the hyperinflammatory response upon *Salmonella* injection, nine CT26 tumor model mice were injected with the strains SHJ2037 and CNC18 and PBS, respectively (three mice per strain or PBS), and the weight of the spleen of each mouse was measured at different time points and compared. As shown in FIG. 11, each mouse was laparotomized, the spleen was removed therefrom, and the size of the spleen of each mouse was measured. The results are shown in FIG. 12.

As shown in FIG. 12, it was confirmed that the strain-treated groups had an increased spleen size compared to the PBS-treated control group, but the strain CNC18-treated group had a smaller spleen size than the strain SHJ2037-treated group, indicating that the *Salmonella* strain according to the present invention minimized the hyperinflammatory reaction.

### [Experimental Example 5] Analysis (2) of hyperinflammatory response upon Salmonella injection

In addition, in order to examine the hyperinflammatory response upon *Salmonella* injection, each of the strains SHJ2037, CNC16, CNC17 and CNC18 was injected to mouse in the same way as described in Experimental Example 4, and the weight of the spleen of each mouse was measured on days 1, 3, and 5 and compared.

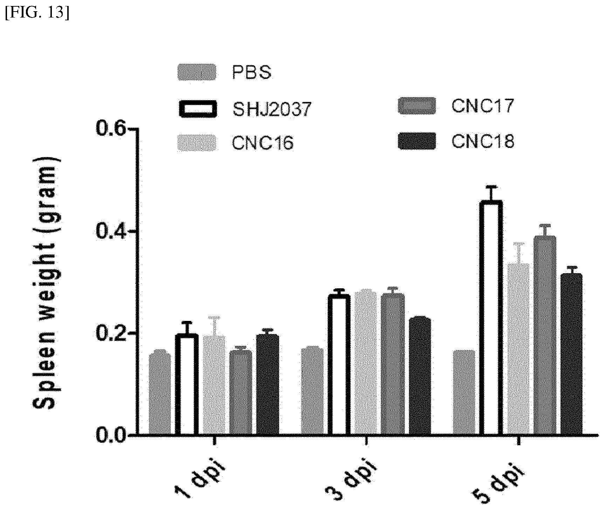

As a result, as shown in FIG. 13, it was confirmed that the group treated with each of the strains SHJ2037, CNC16 and CNC17 had an increased spleen weight compared to the control group treated with PBS, but the CNC18 had the smallest increase in the spleen weight among the strain-treated groups.

From the above results, it was confirmed that the attenuated *Salmonella* strain according to the present invention died rapidly with a significantly decreased viability in all the normal organs from the initial stage of infection while maintaining strong tumor targeting ability, indicating the minimized side effects of the strain on the host.

### [Experimental Example 6] Analysis (1) of anticancer effect of attenuated Salmonella in small animal tumor models

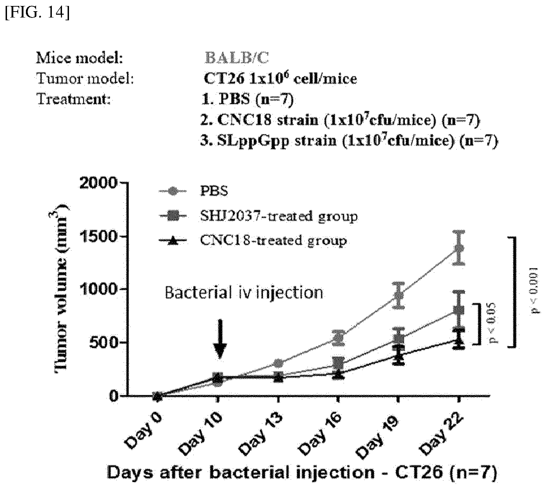

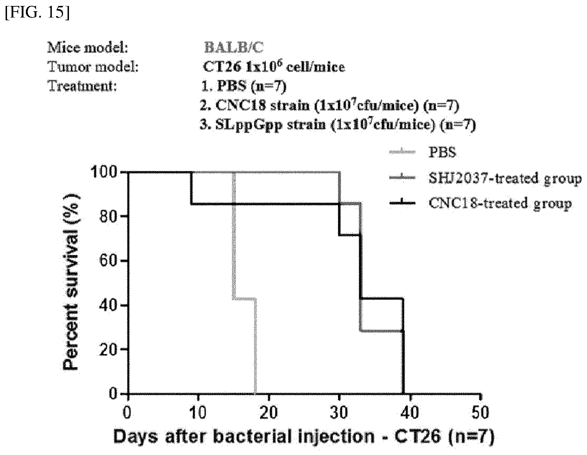

The tumor cells (CT26 1×10⁶ cell/mice) cultured as in Experimental Example 2 were injected subcutaneously into the flanks of mice (BALB/C, n=7), thereby constructing tumor animal models. Each of the *Salmonella* strains SHJ2037 and CNC18 was injected into the tail vein of each of the tumor animal models. To evaluate the anticancer effect of each strain in the tumor animal models, the mice were anesthetized with 2% isoflurane, and then the tumor volume (mm³) was measured using the formula (length X height X width)/2. The results are shown in FIGS. 14 and 15.

As shown in FIGS. 14 and 15, it was confirmed that, compared to the control group, cancer growth in the *Salmonella-treated* group was inhibited and the survival rate of the treated group increased. In particular, it was confirmed that the strain CNC18 was significantly more effective in inhibiting tumor growth than SHJ2037.

Therefore, it was confirmed that the strain CNC18, which dies rapidly with a significantly decreased viability in all normal organs from the initial stage of infection while maintaining strong tumor targeting ability, has excellent tumor treatment efficacy while minimizing side effects caused by infection in the host.

### [Experimental Example 7] Analysis (2) of anticancer effect of attenuated Salmonella in small animal tumor models

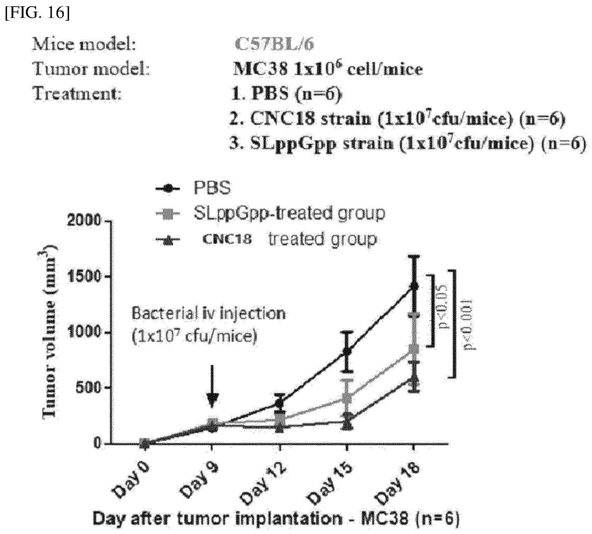

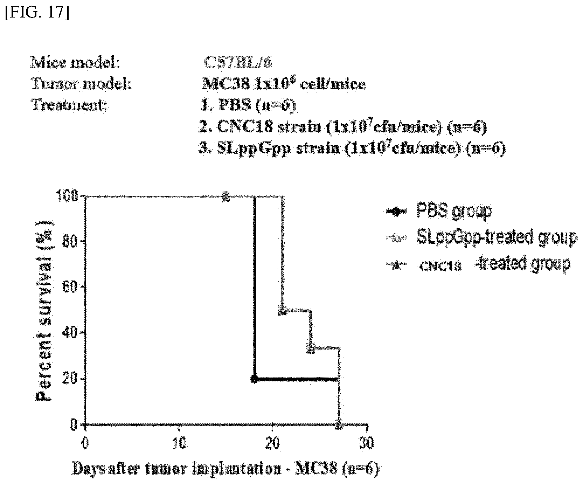

Cultured tumor cells (MC38, murine colon adenocarcinoma cells, 1×10⁶ cell/mice) were injected subcutaneously into the flanks of mice (C57BL/6, n=6), thereby constructing tumor animal models. Each of the *Salmonella* strains SHJ2037 and CNC18 was injected into the tail vein of each of the tumor animal models. To evaluate the anticancer effect of each strain in the tumor animal models, the mice were anesthetized with 2% isoflurane, and then the tumor volume (mm³) was measured using the formula (length X height X width)/2. The results are shown in FIGS. 16 and 17.

As shown in FIGS. 16 and 17, it was confirmed that, compared to the control group, cancer growth in the *Salmonella-treated* group was inhibited and the survival rate of the treated group increased. In particularly, it was confirmed that the strain CNC18 was more effective in inhibiting tumor growth than SHJ2037.

### [Experimental Example 8] Evaluation of immune activation effect of Salmonella injection

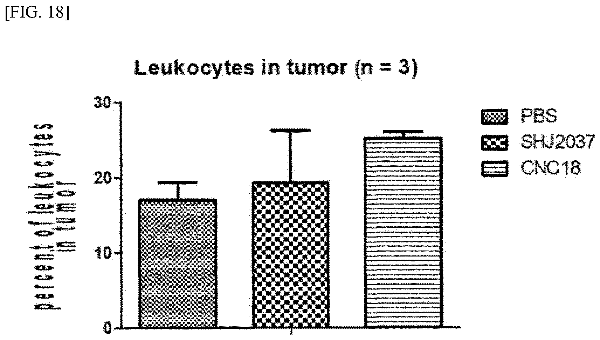

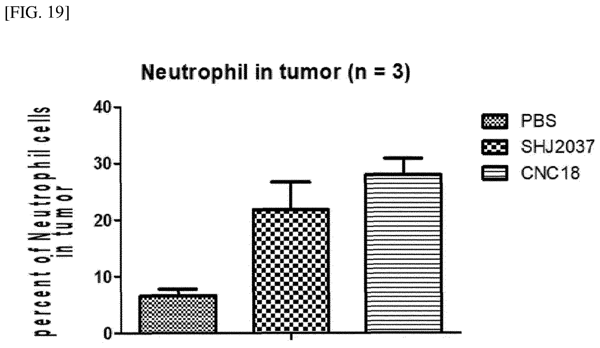

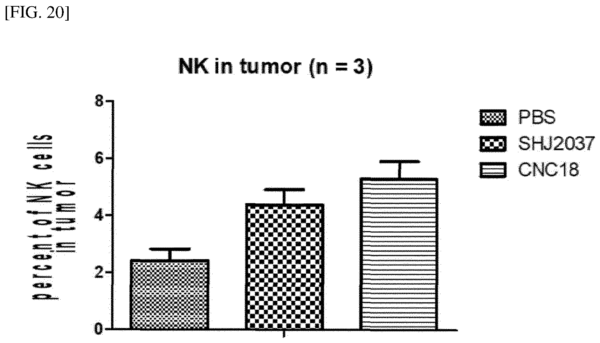

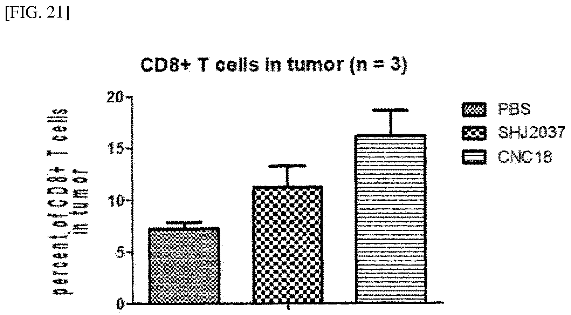

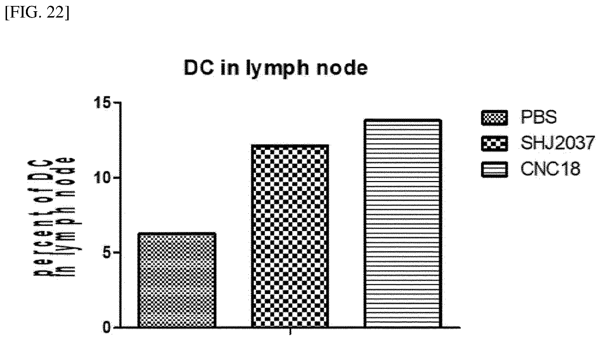

To evaluate the immune activation effect of *Salmonella* injection, immune cells were measured and compared. Each of the *Salmonella* strains SHJ2037 and CNC18 was injected into the tumor animal models constructed described in Experimental Example 6, and immune cells were collected on day 3. The immune cells were collected from a tumor and peri-tumor lymph nodes. The amount of the collected immune cells was measured and the results are shown in FIGS. 18 to 24.

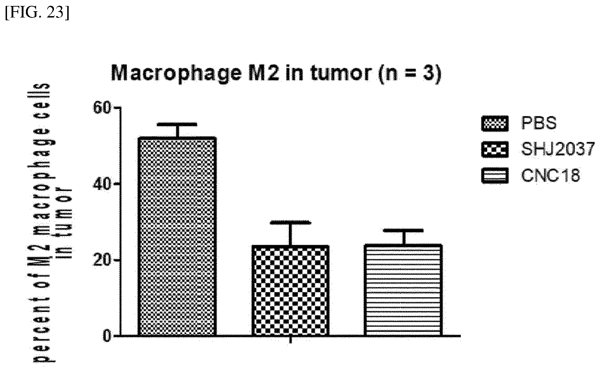

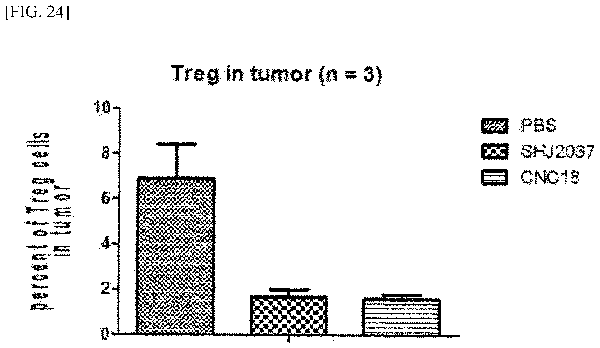

As shown in FIGS. 18 to 22, it was confirmed that the strain CNC18-treated group showed increases in the proportions of leukocytes, neutrophils, natural killer cells and CD8+ T cells compared to the PBS control group and the SHJ2037 control group, and showed an increase in the proportion of dendritic cells in the lymph node. On the other hand, as shown in FIGS. 23 and 24, it was confirmed that the strain CNC18-treated group showed decreases in the proportions of immunosuppressive M2 macrophages and regulatory T (Treg) cells compared to the PBS control group and the SHJ2037 control group.

From the above results, it was confirmed that the attenuated *Salmonella* strain CNC18 according to the present invention had the effect of significantly increasing the immune function compared to the PBS control group and the SHJ2037 control group.

### [Experimental Example 9] Analysis (1) of tumor imaging by Salmonella

The tumor cells cultured as described in Experimental Example 2 were injected subcutaneously into the flanks of mice, thereby constructing tumor animal models. Each of the *Salmonella* strains SHJ2037lux and CNC181ux was injected into these models. Here, the strains SHJ2037lux and CNC18lux were strains into which a luminescence gene has been introduced into the strain SHJ2037 and the strain CNC18. As the luminescence gene, a bacterial luciferase gene (lux) was used.

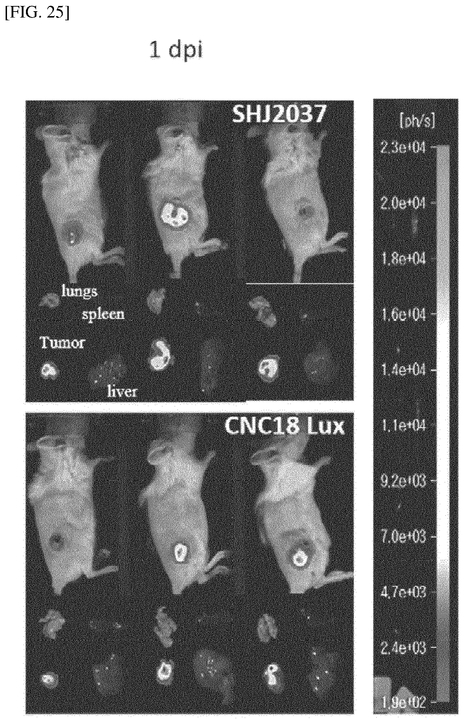

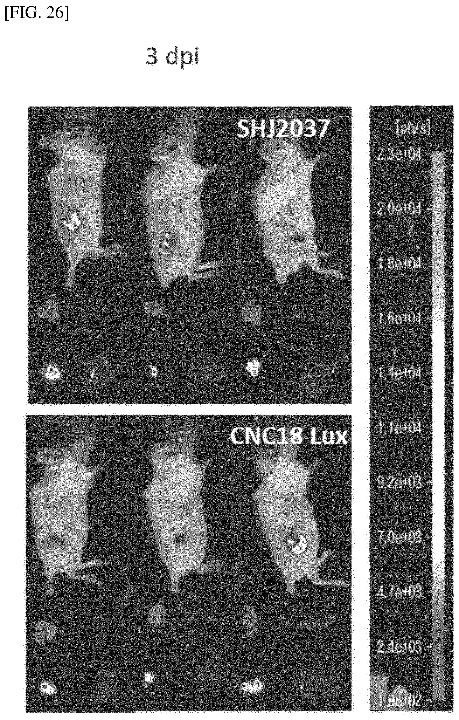

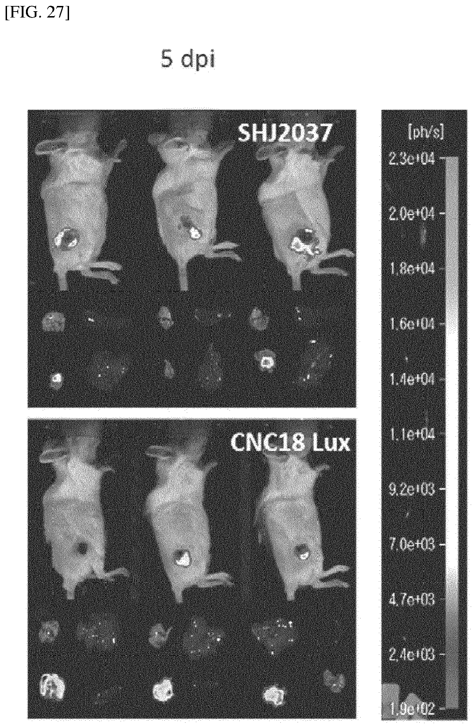

In order to evaluate the imaging effect of each strain in the tumor animal models, organs and tumors were extracted at each time points and then imaged using *in vivo* imaging system (IVIS). The results are shown in FIG. 25 to 27.

As shown in FIGS. 25 to 27, it was confirmed that, like the strain SHJ2037lux, when the strain CNC18lux was injected, it enabled real-time imaging while showing tumor-specific and selective viability.

### [Experimental Example 10] Analysis (2) of tumor imaging by Salmonella

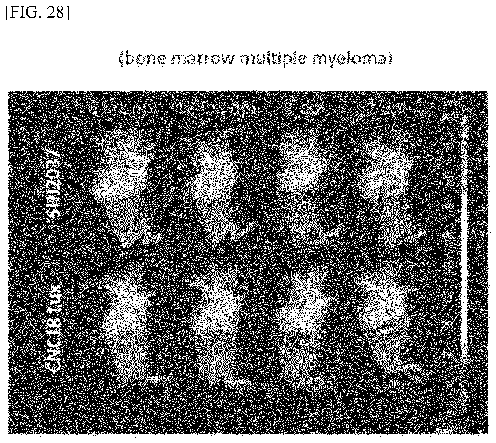

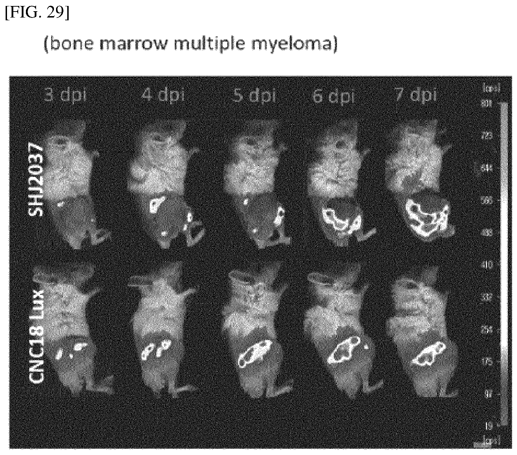

Multiple myeloma models were constructed by injecting cultured MOPC cells into the shinbones of mice, and then each of the *Salmonella* strains SHJ2037lux and CNC18lux was injected into the models in the same manner as described in Experimental Example 9. Next, the mice were imaged at each time points using an *in vivo* imaging system (IVIS), and the results are shown in FIGS. 28 and 29.

As shown in FIGS. 28 and 29, it was confirmed, when the strain CNC181ux was injected, it enabled real-time imaging while showing tumor-specific and selective viability. In particular, it could be confirmed that, unlike the strain SHJ2037lux showing dispersed image signals while remaining in the periphery of the tumor, the strain CNC 181ux reached the deep portion of the tumor, enabling clearer real-time imaging.

As can be seen in Experimental Examples 1 to 10, it was confirmed that the CNC18 strain dies rapidly with a significantly decreased viability in all normal organs from the initial stage of infection while maintaining strong tumor-targeting ability, and exhibits excellent tumor treatment efficacy while minimizing side effects caused by infection in the host.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The present invention is directed to a *Salmonella* strain selectively acting on cancer and a composition for preventing or treating cancer containing the same.

## Claims

1. A *Salmonella* sp. mutant strain in which *Salmonella* pathogenicity island-1 (SPI-1) and *Salmonella* pathogenicity island-2 (SPI-2) are deleted.

2. The *Salmonella* sp. mutant strain according to claim 1, wherein the *Salmonella* pathogenicity island-1 (SPI-1) consists of the nucleotide sequence set forth in SEQ ID NO: 1.

3. The *Salmonella* sp. mutant strain according to claim 1, wherein the *Salmonella* pathogenicity island-2 (SPI-2) consists of the nucleotide sequence set forth in SEQ ID NO: 2.

4. The *Salmonella* sp. mutant strain according to claim 1, wherein the *Salmonella* sp. mutant strain is one into which a gene encoding an anticancer protein has been additionally introduced.

5. The *Salmonella* sp. mutant strain according to claim 4, wherein the anticancer protein is at least one selected from the group consisting of a toxin protein, an antibody specific for a cancer antigen or a fragment of the antibody, a tumor suppressor protein, an angiogenesis inhibitor, a cancer antigen, a prodrug-converting enzyme, and a pro-apoptotic protein.

6. The *Salmonella* sp. mutant strain according to claim 5, wherein the toxin protein is at least one selected from the group consisting of ricin, saporin, gelonin, momordin, debouganin, diphtheria toxin, Pseudomonas toxin, hemolysin (HlyA), FAS ligand (FASL), tumor necrosis factor-α (TNF-α), TNF-related apoptosis-inducing ligand (TRAIL), and cytolysin A (ClyA).

7. The *Salmonella* sp. mutant strain according to claim 6, wherein the cytolysin A consists of the nucleotide sequence set forth in SEQ ID NO: 15.

8. The *Salmonella* sp. mutant strain according to claim 5, wherein the tumor suppressor protein is at least one selected from the group consisting of retinoblastoma (RB) protein, p53 protein, adenomatous polyposis *coli* (APC) protein, phosphatase and tensin homologue (PTEN) protein, and cyclin dependent kinase inhibitor 2A (CDKN2A) protein.

9. The *Salmonella* sp. mutant strain according to claim 5, wherein the angiogenesis inhibitor is at least one selected from the group consisting of angiostatin, endostatin, thrombospondin, and protease inhibitory proteins.

10. The *Salmonella* sp. mutant strain according to claim 5, wherein the cancer antigen is at least one selected from the group consisting of α-fetoprotein (AFP), vascular endothelial growth factor receptor 2 (VEGFR2), Survivin, Legumain, and prostate cancer-specific antigen (PCSA).

11. The *Salmonella* sp. mutant strain according to claim 5, wherein the prodrug-converting enzyme is at least one selected from the group consisting of thymidine kinase, cytosine deaminase, nitroreductase, purine nucleoside phosphorylase, carboxypeptidase G2, chromate reductase YieF, herpes simplex virus type I thymidine kinase/ganciclovir (HSV1-TK/GCV), and β-glucuronidase.

12. The *Salmonella* sp. mutant strain according to claim 5, wherein the pro-apoptotic protein is L-ASNase or RNA-binding motif protein 5 (RBM5).

13. The *Salmonella* sp. mutant strain according to claim 1, wherein the *Salmonella* sp. mutant strain is derived from at least one selected from the group consisting of *Salmonella typhimurium, Salmonella choleraesuis, Salmonella enteritidis, Salmonella infantis, Salmonella paratyphi,* and *Salmonella typhi.*

14. The *Salmonella* sp. mutant strain according to claim 1, wherein the *Salmonella* sp. mutant strain is a mutant strain lacking ability to synthesize guanosine polyphosphate.

15. The *Salmonella* sp. mutant strain according to claim 1, wherein the *Salmonella* sp. mutant strain is at least one in which ppGpp synthase-encoding *Salmonella*-relA gene is inactivated, or *Salmonella-spoT* gene is inactivated.

16. A method for producing a *Salmonella* sp. mutant strain, the method comprising a step of removing *Salmonella* pathogenicity island-1 (SPI-1) and *Salmonella* pathogenicity island-2 (SPI-2) genes from a *Salmonella* sp. strain to obtain a transformed strain.

17. The method according to claim 16, wherein the removing the *Salmonella* pathogenicity island-1 (SPI-1) and the *Salmonella* pathogenicity island-2 (SPI-2) genes is performed by a recombinant vector.

18. The method according to claim 16, wherein the *Salmonella* pathogenicity island-1 (SPI-1) consists of the nucleotide sequence set forth in SEQ ID NO: 1.

19. The method according to claim 16, wherein the *Salmonella* pathogenicity island-2 (SPI-2) consists of the nucleotide sequence set forth in SEQ ID NO: 2.

20. The method according to claim 16, further comprising a step of introducing a gene encoding an anticancer protein.

21. The method according to claim 20, wherein the anticancer protein is at least one selected from the group consisting of ricin, saporin, gelonin, momordin, debouganin, diphtheria toxin, Pseudomonas toxin, hemolysin (HlyA), FAS ligand (FASL), tumor necrosis factor-α (TNF-α), TNF-related apoptosis-inducing ligand (TRAIL), and cytolysin A (ClyA).

22. The method according to claim 21, wherein the gene encoding the cytolysin A consists of the nucleotide sequence set forth in SEQ ID NO: 15.

23. The method according to claim 16, wherein the *Salmonella* sp. strain is at least one selected from the group consisting of *Salmonella typhimurium, Salmonella choleraesuis, Salmonella enteritidis, Salmonella infantis, Salmonella paratyphi,* and *Salmonella typhi.*

24. The method according to claim 16, wherein the *Salmonella* sp. strain is a mutant strain lacking ability to synthesize guanosine polyphosphate.

25. The method according to claim 16, wherein the *Salmonella* sp. strain is one in which ppGpp synthase-encoding *Salmonella*-relA gene or *Salmonella-spoT* gene is inactivated.

26. The method according to claim 16, further comprising a step of culturing the transformed strain.

27. The method according to claim 16, further comprising a step of culturing the transformed strain in an antibiotic medium and selecting the mutant strain.

28. A pharmaceutical composition for preventing or treating cancer, the pharmaceutical composition containing, as an active ingredient, the *Salmonella* sp. mutant strain according to any one of claims 1 to 15.

29. The pharmaceutical composition according to claim 28, wherein the cancer is selected from the group consisting of melanoma, fallopian tube cancer, brain cancer, small intestine cancer, esophageal cancer, lymph adenocarcinoma, gallbladder cancer, blood cancer, thyroid cancer, endocrine adenocarcinoma, oral cancer, liver cancer, biliary tract cancer, colorectal cancer, rectal cancer, cervical cancer, ovarian cancer, kidney cancer, stomach cancer, duodenal cancer, prostate cancer, breast cancer, brain tumor, lung cancer, undifferentiated thyroid cancer, uterine cancer, colon cancer, bladder cancer, ureter cancer, pancreatic cancer, bone/soft tissue sarcoma, skin cancer, non-Hodgkin's lymphoma, Hodgkin's lymphoma, multiple myeloma, leukemia, myelodysplastic syndrome, acute lymphoblastic leukemia, acute myeloid leukemia, chronic lymphocytic leukemia, chronic myelogenous leukemia, and solitary myeloma.

30. A composition for diagnosing cancer, the composition containing, as an active ingredient, the *Salmonella* sp. mutant strain according to any one of claims 1 to 15.

31. A method for providing information for diagnosing cancer, the method comprising a step of treating a biological sample, isolated from a subject of interest, with the strain according to any one of claims 1 to 15.

32. The method according to claim 31, further comprising a step of diagnosing cancer when a reporter protein is expressed from the strain.

33. A method for preventing or treating cancer, the method comprising a step of administering to a subject an effective amount of the *Salmonella* sp. mutant strain according to any one of claims 1 to 15.
